# EUROPEAN PATENT APPLICATION

(11) **EP 4 417 620 A1**
(43) Date of publication of application: **21.08.2024**
(21) Application number: 22881240.0
(22) Date of filing: 22.09.2022
(51) Int. Cl.: C07K 16/00, C07K 16/24

(54) **METHOD FOR PRODUCING ANTIBODY POPULATION**

(30) Priority: 12.10.2021 KR 20210134891
(71) Applicant: Prestige Biologics Co., Ltd., Cheongju-si, Chungcheongbuk-do 28160 (KR)
(72) Inventor: PARK, Joo Yang, Seoul 04968 (KR); JUNG, Kyung Hee, Sejong 30016 (KR); PARK, Ji Sung, Chungcheongbuk-do 28164 (KR); KU, Jin Young, Chungcheongbuk-do 28160 (KR)
(74) Representative: Frenkel, Matthias Alexander
(86) International application number: PCT/KR2022/014143
(87) International publication number: WO 2023/063611

(57) **Abstract**

The present invention relates to a method for producing an antibody population and, specifically, to an antibody population of desired quality that is produced by culturing recombinant cells expressing antibodies under elaborately controlled culturing conditions such as pH and culture temperature, and a method for effectively producing an antibody population of high quality with excellent biological activity. The present invention can effectively prepare an antibody population having desired proportions of main active antibodies and isomeric antibodies and an antibody population having a desired glycan structure by adjusting pH, culture temperature, and/or lactic acid supply. In addition, the method of the present invention can produce high-quality antibodies having excellent biological activity capable of reaching desired therapeutic efficacy when therapeutic monoclonal antibodies are generated. In particular, with respect to the manufacture of biosimilar drugs, it is possible to effectively manufacture antibodies of the same or very similar quality to the original drug by elaborately adjusting the culture conditions.

## Description

### TECHNICAL FIELD

The present invention relates to a method for producing an antibody population. Specifically, the present invention relates to a method for obtaining an antibody population of desired quality by precisely controlling culture conditions when culturing recombinant cells expressing an antibody.

### BACKGROUND ART

According to the Biopharmaceutical Industry Trend Report published by the Korea Biomedicine Industry Association in December 2020, the global biopharmaceutical market has grown at an average annual growth rate of about 8.6% from 2012 to 2019 and is expected to grow at an average annual growth rate of about 10.1% in the future to reach approximately $505 billion dollars in 2026.

In Korea, the expansion of the biopharmaceutical industry is also expected to continue as the population ages and chronic diseases increase, and considering that the biopharmaceutical market size increased by 16.6% from 2.2309 trillion KRW in 2018 to 2.6002 trillion KRW in 2019, the growth is expected to further expand. However, contrary to the increase in the size of the domestic biopharmaceutical market, it was reported that the production and export of domestic biopharmaceuticals decreased by 2.8% and 12.8%, respectively, in 2019 compared to 2018, and accordingly, in response to the growth of the global biopharmaceutical market, active research and development is required to improve the productivity and quality of biopharmaceuticals.

Antibody therapeutics, which are pharmaceuticals that account for 51.1% of global biopharmaceuticals based on sales in 2019, are produced in a bioprocess that involves recombining cells from mammals such as Chinese hamster ovary (CHO) cells and culturing them in a bioreactor suitable for this. This process sensitively reacts to even tiny changes in culture conditions such as medium, temperature, and pH, affecting not only the productivity of an antibody but also the quality of pharmaceuticals using it. Accordingly, in order to obtain high-quality antibodies at maximum yield, research to control various process parameters of culture conditions has continued for a long time, however, since even tiny changes in numerical values result in large changes, and the productivity and antibody quality (N-glycan analysis, etc.) under specific conditions for each specific antibody are significantly different, there have been difficulties in acquiring satisfactory results in this regard.

In addition, in the case of biosimilar drugs, the key is to obtain a product with a quality as similar as possible to the original drug, and the control of the proportions of main active antibodies and isomeric antibodies in an acquired antibody population and a glycan structure are also important factors, therefore establishing optimized culture conditions considering these conditions still remains a technical challenge.

Patent documents related to research aimed at improving the productivity and quality of products by adjusting the culture conditions of recombinant cells include Korea Patent Publication No. KR 10-2021-0043618 (date of publication: April 21, 2021) and Korea Patent Registration No. KR 10-1724405 (date of registration: April 3, 2017), however, there is absolutely nothing known about how to appropriately control the proportions of main active antibodies and isomeric antibodies in an antibody population and a glycan structure thereof while maximizing the productivity and quality of antibodies.

### SUMMARY OF THE INVENTION

### Technical problem

One object of the present invention is to provide a method for producing an antibody population of desired quality and a high-quality antibody population with excellent biological activity by precisely controlling culture conditions when culturing recombinant cells expressing an antibody.

Specifically, another object of the present invention is to provide a method for producing an antibody population having desired proportions of main active antibodies and isomeric antibodies by controlling pH, culture temperature, and/or lactic acid supply when culturing recombinant cells expressing an antibody.

In addition, still another object of the present invention is to provide a method for producing an antibody population having a desired glycan structure by controlling pH, culture temperature, and/or lactic acid supply when culturing recombinant cells expressing an antibody.

The objects of the present invention are not limited to the above description, and the present invention is provided for all cases in which appropriate effects may be obtained by utilizing the present invention.

### Technical Solution

To produce an antibody population having desired quality, the present inventors discovered that the proportions of main active antibodies and isomeric antibodies in an antibody population may be controlled and a glycan structure may be controlled through precise control of pH, culture temperature, and lactic acid supply during recombinant cell culture and completed the present invention based on this.

Specifically, the present invention provides a method for producing an antibody population, including: (a) a first temperature culture step of culturing recombinant cells expressing an antibody in a medium under conditions of pH 7.0 to 7.1 at a first culture temperature of 36 °C to 38 °C for four to six days; and
(b) a second temperature culture step of culturing the cultured recombinant cells in a medium under conditions of pH 7.0 to 7.1 at a second culture temperature set to be 2 °C to 4 °C lower than the first culture temperature of the step (a), but higher than or equal to 34 °C for six to eight days.

In addition, the present invention provides a method for producing an antibody population, wherein in step (b), culture is performed by adding 80 to 120 µl of 0.1 to 2 M lactic acid per 10 ml of a medium every day from the first day, second day or third day of culture at the second culture temperature.

The term "antibody" used in the present invention refers to a substance produced by stimulation of an antigen in an immune system, which is present in lymph and blood and specifically binds to a specific antigen to cause an antigen-antibody reaction. In the present invention, the type of antibody is not particularly limited and may include all antibodies commonly known in the art and commonly used therapeutic antibodies. For the purpose of the present invention, the antibody may be a monoclonal antibody, and as a more specific example, it may be adalimumab.

The term "monoclonal antibody" used in the present invention refers to an antibody that reacts only with a single epitope and is produced from a single antibody-producing cell.

The term "adalimumab" used in the present invention is a monoclonal antibody against TNF-α, and it is known to be effective in treating rheumatoid arthritis, psoriatic arthritis, ankylosing spondylitis, Crohn's disease, ulcerative colitis, psoriasis, hidradenitis suppurativa, uveitis, juvenile idiopathic arthritis, etc. Adalimumab is a component of the drug Humira^{®} developed by AbbVie, a company in the United States, and is distributed as a therapeutic agent for autoimmune diseases.

The term "recombinant cells expressing an antibody" used in the present invention refers to cells that are recombined to produce a specific antibody through transformation, and their type is not particularly limited. A non-limiting example of the recombinant cell may be a mammalian cell, and more specifically, it may be a Chinese hamster ovary cell (CHO).

The "recombinant cells expressing an antibody" of the present invention may be recombinant cells expressing a monoclonal antibody for the purpose of the present invention, and more specifically, they may be recombinant cells expressing adalimumab. In one embodiment of the present invention, recombinant CHO cells expressing adalimumab were used.

The term "medium" used in the present invention broadly refers to a nutrient-containing solution that supplies nutrients to proliferating cells, and such a solution generally includes essential and non-essential amino acids, vitamins, carbon sources, lipids, and trace elements that are necessary for cells to proliferate and/or survive, but it is not limited thereto. The medium is preferably prepared at a pH and salt concentration that are optimal for cell survival and proliferation depending on the type of cells to be cultured, and it may further include substances that are widely used in the art as ingredients that enhance proliferation and/or survival, including hormones and growth factors.

In the present invention, the method for producing an antibody population may include steps divided into a first temperature culture step and a second temperature culture step depending on culture temperature conditions for culturing recombinant cells expressing an antibody. The first temperature culture step may be performed by culturing recombinant cells expressing an antibody in a medium under conditions of pH 7.0 to 7.1 at a first culture temperature of 36 °C to 38 °C for four to six days. In the first temperature culture step, for the purpose of the present invention, to produce a high-quality adalimumab antibody population, a pH range may be set to be pH 7.0 to 7.1, more specifically around pH 7.0, and more specifically pH 7.0, a first culture temperature range may be set to 36 °C to 38 °C, more specifically 36.5 °C to 37.5 °C, and more specifically around 37 °C, and a culture period may be set to be four to six days, more specifically, five days.

The second temperature culture step may be performed by culturing the recombinant cells cultured in the first temperature culture step in a medium under conditions of pH 7.0 to 7.1 at a second culture temperature set to be 2 °C to 4 °C lower than the first culture temperature of the first temperature culture step but higher than or equal to 34 °C for six to eight days. In the second temperature culture step, for the purpose of the present invention, to produce a high-quality monoclonal antibody population, for example, a high-quality adalimumab antibody population, a pH range may be set to be pH 7.0 to 7.1, more specifically around pH 7.0, and more specifically pH 7.0, a second culture temperature range may be set to 34 °C to 36 °C, more specifically 34.5 °C to 35.5 °C, and more specifically around 35 °C, and a culture period may be set to be six to eight days, more specifically, seven days.

In addition, in the present invention, the second temperature culture step may be performed by adding 80 to 120 µl of 0.1 to 2 M lactic acid per 10 ml of a medium every day from the first day, second day or third day of culture at the second culture temperature. When the second temperature culture step is performed by adding lactic acid, for the purpose of the present invention, to produce a high-quality monoclonal antibody population, for example, a high-quality adalimumab antibody population, the timing of lactic acid addition may be from the first day, second day or third day of culture at the second culture temperature, more specifically, from the second day of culture, more specifically, from the seventh day of total culture, which is from the second day of culture in the second culture temperature step following five days of culture in the first culture temperature step, and an amount of lactic acid added may be set to be 80 to 120 µl every day, more specifically 90 to 110 µl, and more specifically around 100 µl.

In adding the lactic acid, the meaning of "per 10 ml of a medium" is per 10 ml of working volume (WV) including a medium and a culture solution in a culture vessel on the first to third days of culture in the second temperature culture step, and the meaning of "WV" is something that can be clearly understood by those of ordinary skill in the art.

The form of the added lactic acid is not particularly limited, and any form of lactic acid commonly used in the art can be used. As a non-limiting example, for the purpose of the present invention, lactic acid may be used in the form of a lactic acid solution, more specifically, it may be used in the form of an L-lactic acid solution, and the concentration may be 0.1 to 2 M, more specifically 0.5 to 2 M, and more specifically, around 1 M.

For the purpose of the present invention, to produce a monoclonal antibody population with excellent biological activity, for example, a high-quality adalimumab antibody population, an antibody population was produced in one embodiment by a method including:
(a) a first temperature culture step of culturing recombinant cells expressing an adalimumab antibody in a culture medium under conditions of 37 °C and pH 7.0 to 7.1 for five days; and
(b) a second temperature culture step of culturing the recombinant cells cultured for five days at a culture temperature of 35 °C under conditions of pH 7.0 to 7.1 for seven days while adding 100 µl of a 1 M L-lactic acid solution to a medium every day from the third day of culture.

In one embodiment of the present invention, an adalimumab antibody population was produced in the above manner, thereby producing a high-quality antibody population, specifically an antibody population with very similar quality to Humira, which is a reference drug for adalimumab, including 55% or more, more specifically 60% or more of the main active antibody, and 45% or less, more specifically 40% or less of the isomeric antibody (Example 3). In addition, an adalimumab antibody population was produced in the above manner, thereby producing a high-quality antibody population, specifically an antibody population with very similar quality to Humira, which is a reference drug for adalimumab, having glycan structures including 65% to 80% of the G0F form, 3% to 6% of the GOF+GN form, and 3% to 6% of the GOF-N form (Example 4).

The term "an antibody population" used in the present invention refers to an antibody population including main active antibodies, isomeric antibodies, or both main active antibodies and isomeric antibodies. For the purpose of the present invention, the antibody population may include both main active antibodies and isomeric antibodies, and the isomeric antibodies may include both acidic isomeric antibodies and basic isomeric antibodies.

The term "main active antibody" used in the present invention is an antibody with the highest biological activity among antibodies in an antibody population, meaning that amino acids have not been modified by deamination or oxidation, or the like. The proportion of main active antibodies is a particularly important factor in the quality of antibody therapeutics.

The term "isomeric antibody" used in the present invention refers to an antibody whose biological activity is reduced due to some amino acids in the antibody being modified by deamination or oxidation, or the like, among antibodies of an antibody population. Isomeric antibodies include acidic isomers and basic isomers, and there is a slight difference in charge between main active antibodies, acidic isomer antibodies, and basic isomer antibodies, so they are also referred to as charge variants. Isomeric antibodies may be separated using this charge difference. Non-limiting examples of the isomeric antibody include an isomeric antibody in which asparagine, an amino acid, is deaminated to form aspartate and an isomeric antibody in which methionine, an amino acid, is oxidized to form methionine sulfate. In addition, examples of the isomeric antibody also include an isomeric antibody in which glutamate is modified into pyroglutamate by forming a pentagonal ring structure when glutamate is present at an N-terminus of a heavy chain.

The isomeric antibodies are included in a host cell culture medium at a high proportion when antibodies are produced in host cells such as CHO cells and may be removed through a process such as chromatography and included in an antibody population at a desired proportion. To produce a high-quality antibody population in host cells into which a vector that includes a polynucleotide encoding an antibody has been introduced, it is necessary to include the main active antibody and isomeric antibody as described above in a desired amount.

The isomeric antibodies are those in which some amino acids in the antibody have been modified by deamination or oxidation, and it is known that the biological activity of each isomeric antibody is different, therefore maintaining a constant content distribution of the isomeric antibodies is important for maintaining consistent quality. In particular, in the production of biosimilar antibodies, it is important to produce them so that the proportions of main active antibodies and isomeric antibodies are adjusted and included at a composition that corresponds to or is similar to an original drug (reference drug), and the proportions of main active antibodies and isomeric antibodies may be an important judgment criterion in comparing quality with the reference drug and proving homogeneity etc.

An antibody population produced by the method for producing an antibody population of the present invention may include both main active antibodies and isomeric antibodies, and for the purpose of the present invention, the antibody population may include 50% or more of the main active antibody and 50% or less of the isomeric antibody. In addition, to produce an antibody population having better biological activity, through the method for producing an antibody population of the present invention, an antibody population including 55% or more of the main active antibody and 45% or less of the isomeric antibody may be produced, and an antibody population including 60% or more of the main active antibody and 40% or less of the isomeric antibody may be produced.

In addition, for the purpose of the present invention, an adalimumab antibody population may be produced, and Humira^{®}, the original drug of adalimumab, may be used as a reference drug to produce an antibody population having proportions of main active antibodies and isomeric antibodies similar to the reference drug. To produce a biosimilar drug with quality more similar to that of the reference drug, the antibody population may be: an antibody population including 55% or more of the main active antibody and 45% or less of the isomeric antibody; an antibody population including 55% or more of the main active antibody, 15% or less of the acidic isomeric antibody, and 30% or less of the basic isomeric antibody; an antibody population including 55% or more of the main active antibody, 20% or less of the acidic isomeric antibody, and 25% or less of the basic isomeric antibody; an antibody population including 60% or more of the main active antibody and 40% or less of the isomeric antibody; or an antibody population including 60% or more of the main active antibody, 20% or less of the acidic isomeric antibody, and 20% or less of the basic isomeric antibody; and according to one embodiment of the present invention, the antibody population may be an antibody population including 60% or more of the main active antibody, 20% or less of the acidic isomeric antibody, and 25% or less of the basic isomeric antibody.

To obtain an antibody population with higher purity, the method for producing an antibody population according to the present invention may further include a step of removing impurities such host cell protein (HCP), host cell-derived DNA (HCD), and factors for cell growth after obtaining a mixed sample including an antibody population through culture. A method for removing the impurities is not particularly limited and may be carried out by methods commonly used in the art.

When an antibody population is produced by the method for producing an antibody population according to the present invention, after the impurities are removed, a separate filtration and purification process may be performed to more precisely control the main active antibodies and isomeric antibodies to be included at desired proportions, however, when produced according to the method for producing an antibody population of the present invention, even when a separate filtration and purification process other than the removal of impurities commonly performed in the art is not performed, due to culture condition control and synergistic effects according to pH, culture temperature, and lactic acid supply, an antibody population including main active antibodies and isomeric antibodies at proportions close to the proportions at which the biological activity of a desired antibody, for example, a monoclonal antibody, as a more specific example, adalimumab, may be maximized may be produced. In addition, an antibody population including antibodies and isomeric antibodies at the same or very similar proportions compared to the reference drug adalimumab may be produced.

In the present invention, a method of measuring the proportions of main active antibodies and isomeric antibodies in an antibody population is not particularly limited, and it may be performed by a method commonly used in the art. As a non-limiting example, in one embodiment of the present invention, the proportions of main active antibodies and isomeric antibodies was measured using LapChip^{®} and a cation-exchange high-performance liquid chromatography (CE-HPLC) method.

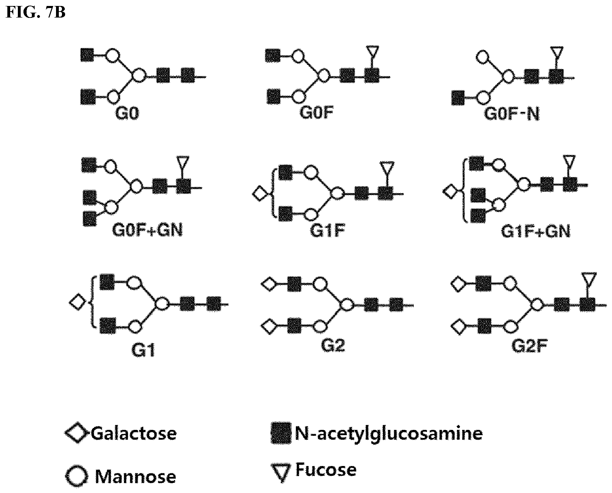

The term "glycan structure" used in the present invention refers to the sugar chain structure caused by fucosylation, afucosylation, galactosylation, etc. of an Fc region of an antibody, and the main types of glycan structures include G0, G0F, GOF-N, GOF+GN, G1F, G1F+GN, G1, G2, and G2F (FIG. 7B).

Since it is known that the biological activity of an antibody varies depending on the glycan structure, and in the case of therapeutic antibodies, having an appropriate glycan structure is an important factor in the quality of drugs. In particular, in the production of biosimilar antibodies, it is important to produce them to have a glycan structure that corresponds to or is similar to that of an original drug (reference drug), and a glycan structure may be an important judgment criterion in comparing quality with the reference drug and proving homogeneity etc.

The method for producing an antibody population of the present invention may allow an antibody population having various glycan structures to be produced according to the purpose, specifically the quality of an antibody population to be obtained. For the purpose of the present invention, by precisely controlling pH, culture temperature, and lactic acid supply, an antibody population having a glycan structure including 65% to 80% of the G0F form, an antibody population having a glycan structure including 3% to 6% of the GOF+GN form, and an antibody population having a glycan structure including 3% to 6% of the GOF-N form may be produced.

In addition, for the purpose of the present invention, an adalimumab antibody population may be prepared, and by using Humira^{®}, the original drug of adalimumab, as a reference drug, an antibody population having a glycan structure similar to that of the reference drug may be produced.

The term "fucosylation" used in the present invention refers to the attachment of a fucose residue to an N-glycan, O-glycan, and glycolipid. The regulation of fucosylation or afucosylation is known to have a significant impact on the function of monoclonal antibodies, and it is known that reduced fucose in a glycosylation pattern, that is, an afucosylated monoclonal antibody, exhibits higher antibody dependent cell-mediated cytotoxicity (ADCC) than a fucosylated antibody.

The term "afucosylation" used in the present invention has the opposite meaning of fucosylation and means the detachment of a fucose residue. This may be used in the same meaning as defucosylation, and it specifically means that the degree of fucosylation is reduced.

In the present invention, a method for analyzing the glycan structure of an antibody and a method for performing N-glycan analysis are not particularly limited and may be performed by methods commonly used in the art. As a non-limiting example, they may be performed using a quadrupole time of flight (Qtof) mass spectrometry analysis method.

Terms that are not otherwise defined in the present invention are construed as having meanings commonly used in the art. In addition, the expression "or" described in the present invention may be interpreted as a concept including "and" unless otherwise specified.

The scope of the present invention is not limited by the specific description disclosed in the present invention, and each description and embodiment disclosed in the present invention may be applied to each different description and embodiment. In other words, all possible combinations of the various elements disclosed in the present invention are to be construed as falling within the scope of the present invention. In addition, those skilled in the art may recognize or confirm many equivalents to specific embodiments of the present invention through common experimentation, and such equivalents are to be construed as falling within the scope of the present invention.

### BENEFIT(S) OF THE INVENTION

A method for producing an antibody population of the present invention is capable of producing an antibody population of desired quality and a high-quality antibody population with excellent biological activity by precisely controlling culture conditions such as pH and culture temperature when culturing recombinant cells.

Specifically, the present invention is capable of effectively producing an antibody population having desired proportions of main active antibodies and isomeric antibodies by controlling pH, culture temperature, and/or lactic acid supply when culturing recombinant cells expressing an antibody.

In addition, the present invention is capable of effectively producing an antibody population having a desired glycan structure by controlling pH, culture temperature, and/or lactic acid supply when culturing recombinant cells expressing an antibody.

In addition, high-quality antibodies having excellent biological activity that can reach the desired therapeutic efficacy can be produced by the method of the present invention, and especially in manufacture of biosimilar drugs, antibodies of the same or very similar quality to that of an original drug can be effectively produced by precisely controlling pH, culture temperature, and/or lactic acid supply conditions.

### BRIEF DESCRIPTION OF THE DRAWINGS

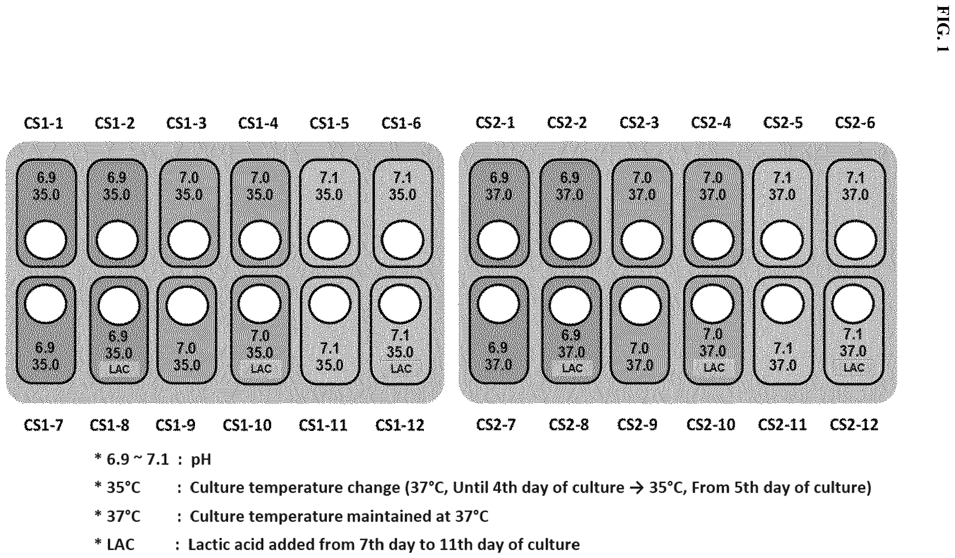

FIG. 1 schematically shows the culture conditions according to Example 1 of the present invention.

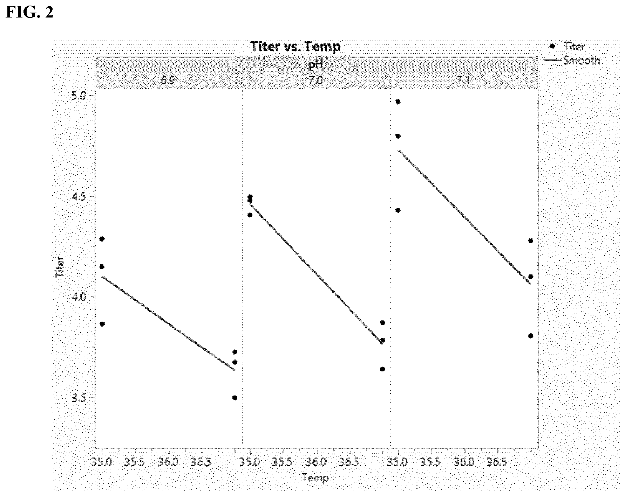

FIG. 2 shows the results of IgG titer measurement according to Example 2 of the present invention.

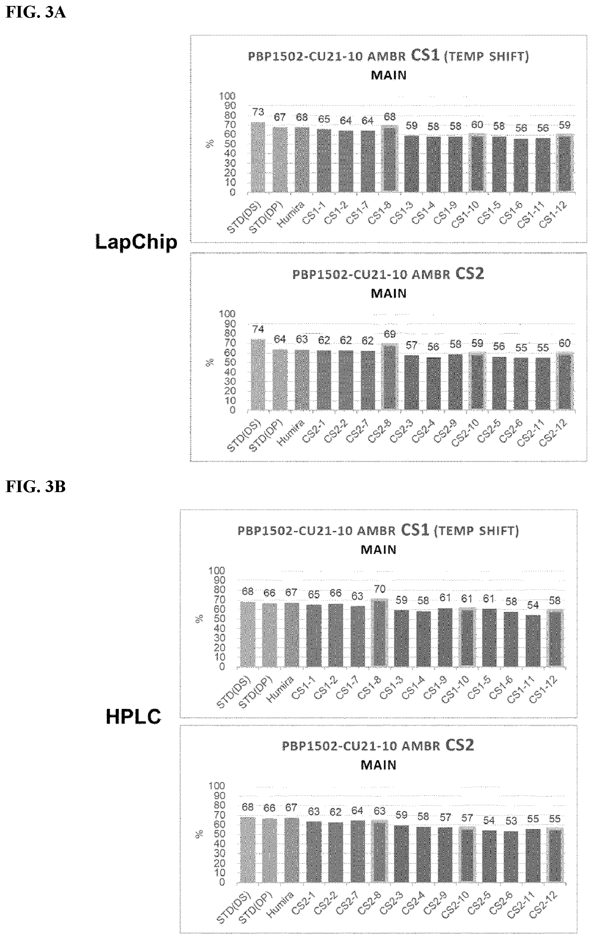

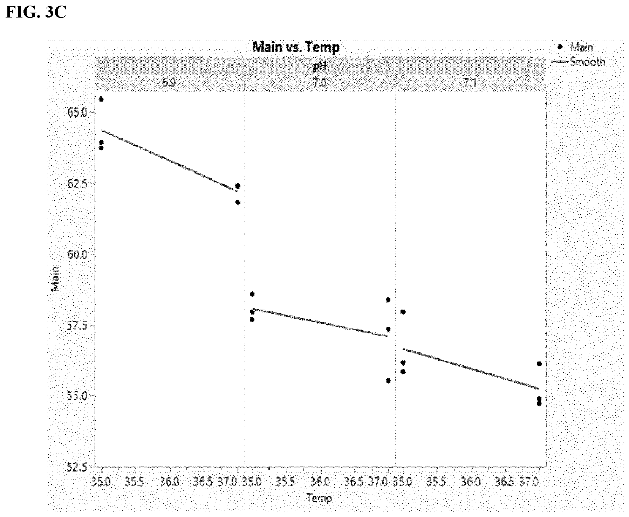

FIGS. 3A to 3C show the results of charge variant analysis using LapChip^{®} and CE-HPLC equipment for the proportion of main active antibodies according to Example 3-1 of the present invention. Specifically, FIG. 3A is a graph showing the results of charge variant analysis using LapChip^{®} equipment, FIG. 3B is a graph showing the results of charge variant analysis using CE-HPLC equipment, and FIG. 3C is a graph comprehensively showing the proportion of main active antibodies according to pH and temperature. The name and color of each column in the graphs of FIGS. 3A and 3B are the same as the indications schematically showing the culture conditions in FIG. 1.

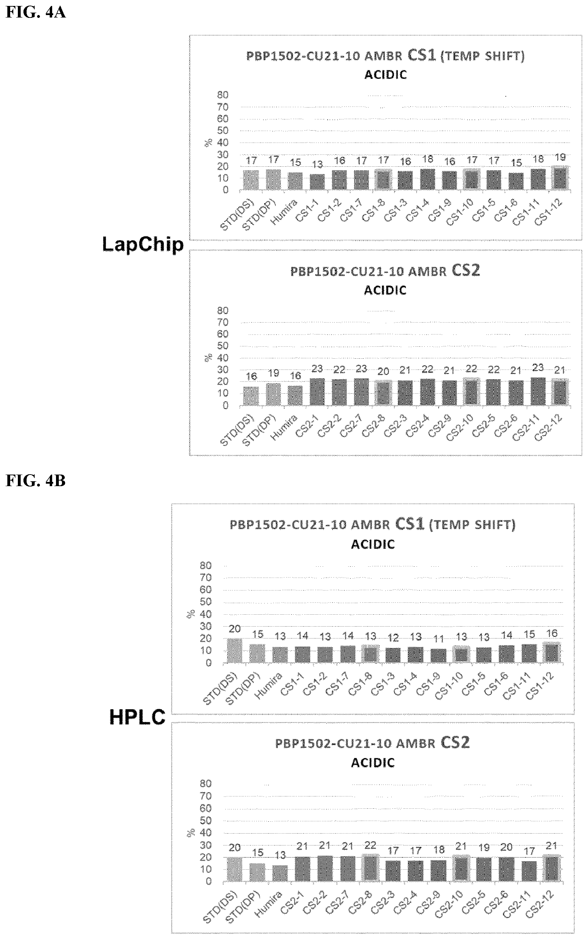

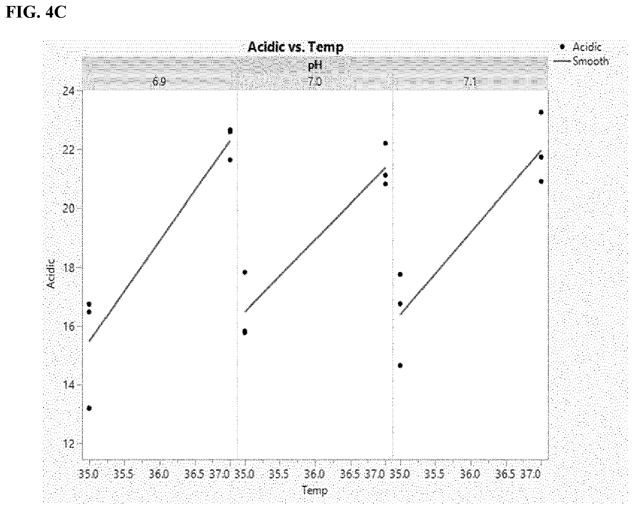

FIG. 4A to 4C show the results of charge variant analysis using LapChip^{®} and CE-HPLC equipment for the proportion of acidic isomeric antibodies according to Example 3-2 of the present invention. Specifically, FIG. 4A is a graph showing the results of charge variant analysis using LapChip^{®} equipment, FIG. 4B is a graph showing the results of charge variant analysis using CE-HPLC equipment, and FIG. 4C is a graph comprehensively showing the proportion of acidic isomeric antibodies according to pH and temperature. The name and color of each column in the graphs of FIGS. 4A and 4B are the same as the indications schematically showing the culture conditions in FIG. 1.

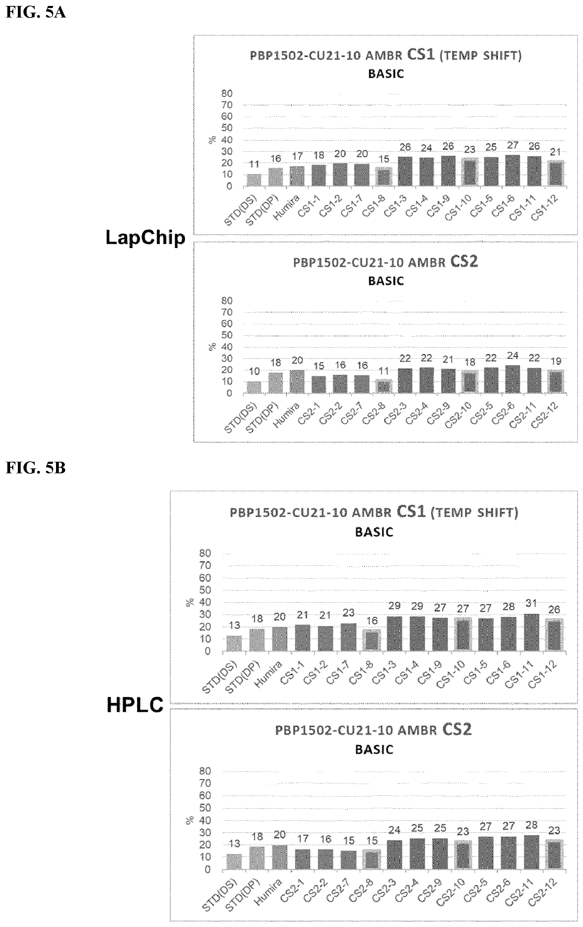

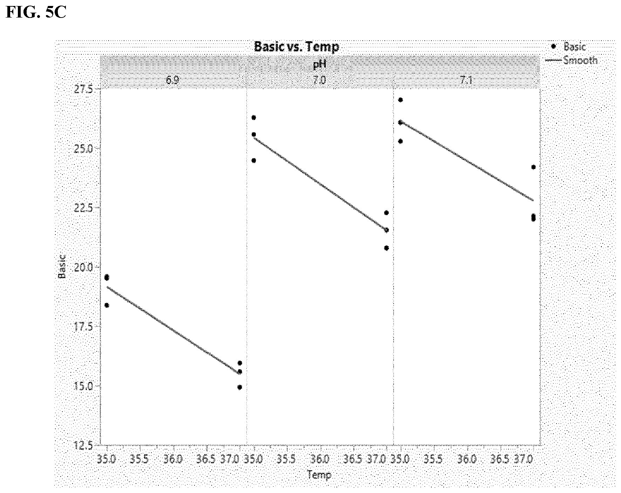

FIG. 5A to 5C show the results of charge variant analysis for the proportion of basic isomeric antibodies according to Example 3-3 of the present invention. Specifically, FIG. 5A is a graph showing the results of charge variant analysis using LapChip^{®} equipment, FIG. 5B is a graph showing the results of charge variant analysis using CE-HPLC equipment, and FIG. 5C is a graph comprehensively showing the proportion of basic isomeric antibodies according to pH and temperature. The name and color of each column in the graphs of FIGS. 5A and 5B are the same as the indications schematically showing the culture conditions in FIG. 1.

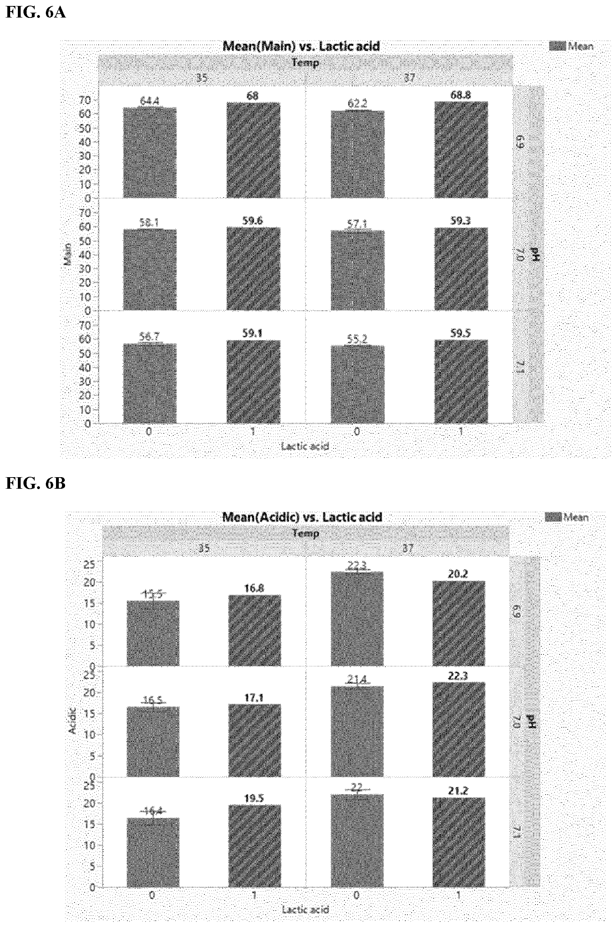

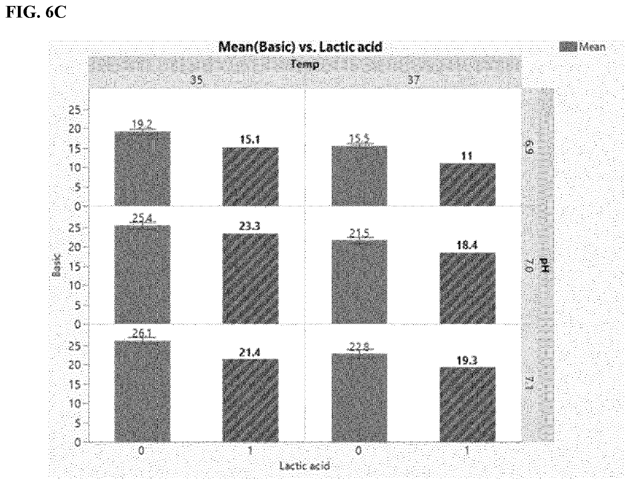

FIG. 6A to 6C are graphs showing the results of charge variant analysis using LapChip^{®} equipment for changes in the proportions of main active antibodies and isomeric antibodies due to lactic acid supply according to Example 3-4 of the present invention. Specifically, FIG. 6A is a graph showing changes in the proportion of main active antibodies, FIG. 6B is a graph showing changes in the proportion of acidic isomeric antibodies, and FIG. 6C is a graph showing changes in the proportion of basic antibodies.

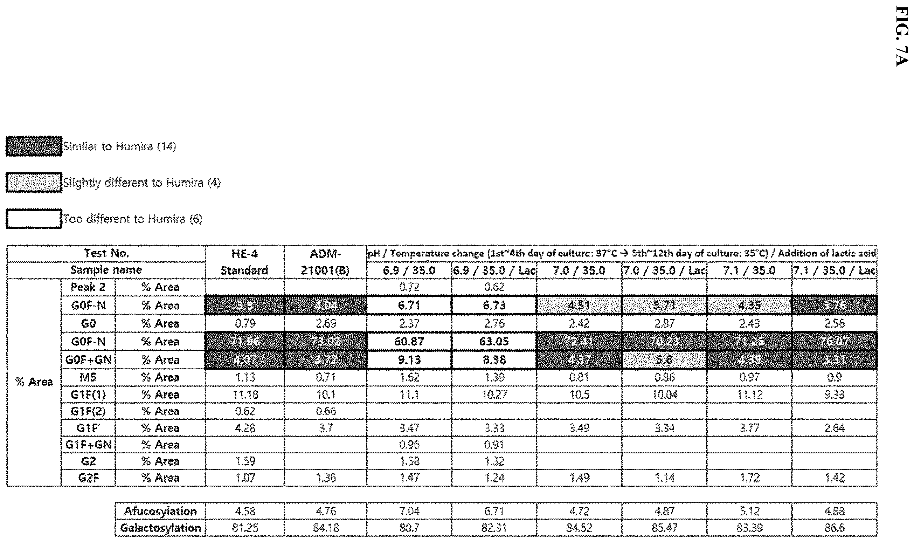

FIG. 7A and 7B show the results of N-glycan analysis according to Example 4 of the present invention. Specifically, FIG. 7A is a table showing the results of N-glycan analysis of an obtained antibody population and the results of similarity analysis with a control drug (Humira), and FIG. 7B schematically shows a glycan structure used in glycan analysis.

### DETAILED DESCRIPTION

Hereinafter, the present invention will be described in more detail through specific examples. However, these examples are merely examples for explaining the present invention, and the scope of the present invention should not be construed as being limited in any way by these examples.

### Example 1: Production of antibody population according to pH, temperature, and lactic acid supply control

A culture solution obtained by culturing recombinant Chinese hamster ovary (CHO) cells expressing an adalimumab antibody in a medium was inoculated in an amount of 1.1 mL into 24 disposable culture vessels, each with a volume of 15 mL, and the culture solution was cultured for a total of 12 days in an Ambr^{®}15 bioreactor of Sartorius under the culture conditions shown in Table 1 below.

**[Table 1]**

| Culture vessel | | Temperature (°C) | pH | Lactic acid supply |
|---|---|---|---|---|
| CS1 | 1, 2, 7 | 37.0 → 35.0 (temperature changed on the fifth day of culture) | 6.9 | X |
| | 8 | | | O |
| | 3, 4, 9 | | 7.0 | X |
| | 10 | | | O |
| | 5, 6, 11 | | 7.1 | X |
| | 12 | | | O |
| CS2 | 1, 2, 7 | 37.0 | 6.9 | X |
| | 8 | | | O |
| | 3, 4, 9 | | 7.0 | X |
| | 10 | | | O |
| | 5, 6, 11 | | 7.1 | X |
| | 12 | | | O |

Specifically, 12 of the 24 culture vessels were assigned to a group in which the temperature was changed during culture (CS1-1 to CS1-12), and the other 12 culture vessels were assigned to a group in which the temperature was not changed (CS2-1 to CS2-12), and each group was further divided into three subgroups, which were respectively cultured under conditions of pH 6.9, pH 7.0, and pH 7.1. In addition, one culture vessel per subgroup under each pH condition was cultured by injecting 100 µl of 1 M L-lactic acid solution per 10 ml of working volume (WV) every day from the 7th to the 11th day of culture. Each culture vessel according to the above culture conditions is schematically shown in FIG. 1.

After completion of the culture, the culture medium was purified using a Protein A column, and then IgG titer measurement, LapChip^{®} and cation exchange high-performance liquid chromatography (CE-HPLC) analyses, and N-glycan analysis were performed.

### Example 2: IgG titer measurement

The IgG titer of the antibody populations obtained in Example 1 was measured, and the results are shown in FIG. 2.

As a result of the measurement, it was confirmed that the IgG titer of the culture medium was significantly affected by both culture temperature and pH conditions, and in particular, it was confirmed that the IgG titer was more affected by temperature than pH. In addition, within a certain range, the lower the temperature and the higher the pH, the higher the IgG titer. Accordingly, it was confirmed that conditions of a relatively high pH of 7.0 to 7.1 and conditions of applying a temperature change of lowering the culture temperature from the initial temperature of 37 °C to 35 °C were advantageous for increasing antibody productivity of the recombinant cells (FIG. 2).

### Example 3: LapChip^{®} and CE-HPLC analysis

Charge variant analysis was performed on the antibody populations obtained in Example 1 using LapChip^{®} and CE-HPLC equipment, and the results are shown in FIGS. 3 to 5.

### Example 3-1: Results of main active antibody analysis

The results of charge variant analysis using LapChip^{®} and CE-HPLC equipment are shown in FIG. 3. Specifically, FIG. 3A is a graph showing the results of charge variant analysis using LapChip^{®} equipment, FIG. 3B is a graph showing the results of charge variant analysis using CE-HPLC equipment, and FIG. 3C is a graph comprehensively showing the proportion of main active antibodies according to pH and temperature. The name and color of each column in the graphs of FIGS. 3A and 3B are the same as the indications schematically showing the culture conditions in Example 1 and FIG. 1. Specifically, the upper graphs of each of FIGS. 3A and 3B are graphs for the CS1 group that was cultured at a temperature of 37 °C from the 1st day to the 4th day of culture and then subjected to a temperature change to 35°C from the 5th day of culture, and the lower graphs are for the CS2 group that was maintained at 37 °C without a change in the culture temperature. The orange column on the graphs is a graph for pH 6.9 conditions; the green column is a graph for pH 7.0 conditions; and the blue column is a graph for pH 7.1 conditions, and the three columns outlined with yellow lines are graphs for conditions in which lactic acid was supplied from the 7th day to the 11th day of culture.

As a result of the charge variant analysis using the LapChip^{®} and CE-HPLC equipment, when compared under the same pH conditions, it was confirmed that the CS1 group that was subjected to a temperature change from 37 °C to 35 °C on the 5th day of culture generally exhibited a higher proportion of main active antibodies compared to the CS2 group that was not subjected to a temperature change. In addition, it was confirmed that within a certain range, the lower the pH, the higher the proportion of main active antibodies, and it was confirmed that under the same pH conditions, the proportion of main active antibodies was higher when lactic acid was added to the medium from the 7th day of culture (FIGS. 3A and 3B).

The results for the overall effect of pH and temperature conditions on the proportion of main active antibodies are shown in FIG. 3C, and specifically, it was found that pH and temperature changes have a significant effect on the proportion of main active antibodies, and in particular, it was confirmed that pH conditions have a greater effect on the proportion of main active antibodies than temperature conditions. In addition, it was confirmed that within a certain range, the lower the pH and the lower the temperature, the higher the proportion of main active antibodies (FIG. 3C).

### Example 3-2: Results of acidic isomer antibody analysis

The results of charge variant analysis using LapChip^{®} and CE-HPLC equipment are shown in FIG. 4. Specifically, FIG. 4A is a graph showing the results of charge variant analysis using LapChip^{®} equipment, FIG. 4B is a graph showing the results of charge variant analysis using CE-HPLC equipment, and FIG. 4C is a graph comprehensively showing the proportion of acidic isomeric antibodies according to pH and temperature. The name and color of each column in the graphs of FIGS. 4A and 4B are the same as the indications schematically showing the culture conditions in FIG. 1, and the specific details are the same as described in Example 3-1.

As a result of the charge variant analysis using the LapChip^{®} and CE-HPLC equipment, when compared under the same pH conditions, it was confirmed that the CS1 group that was subjected to a temperature change from 37 °C to 35 °C on the 5th day of culture generally exhibited a lower proportion of acidic isomeric antibodies compared to the CS2 group that was not subjected to a temperature change (FIGS. 4A and 4B).

The results for the overall effect of pH and temperature conditions on the proportion of acidic isomeric antibodies are shown in FIG. 4C. Specifically, it was found that temperature changes have a greater effect on the proportion of acidic isomeric antibodies than pH changes, and it was confirmed that within a certain range, the lower the temperature, the lower the proportion of acidic isomeric antibodies (FIG. 4C).

### Example 3-3: Result of basic isomeric antibody analysis

The results of charge variant analysis using LapChip^{®} and CE-HPLC equipment are shown in FIG. 5. Specifically, FIG. 5A is a graph showing the results of charge variant analysis using LapChip^{®} equipment, FIG. 5B is a graph showing the results of charge variant analysis using CE-HPLC equipment, and FIG. 5C is a graph comprehensively showing the proportion of basic isomeric antibodies according to pH and temperature. The name and color of each column in the graphs of FIGS. 5A and 5B are the same as the indications schematically showing the culture conditions in FIG. 1, and the specific details are the same as described in Example 3-1.

As a result of the charge variant analysis using the LapChip^{®} and CE-HPLC equipment, when compared under the same pH conditions, it was confirmed that the CS1 group that was subjected to a temperature change from 37 °C to 35 °C on the 5th day of culture generally exhibited a lower proportion of basic isomeric antibodies compared to the CS2 group that was not subjected to a temperature change. In addition, it was confirmed that within a certain range, the lower the pH, the lower the proportion of basic isomeric antibodies, and it was confirmed that under the same pH conditions, the proportion of basic isomeric antibodies was lower when lactic acid was added to the medium from the 7th day of culture (FIGS. 5A and 5B).

The results for the overall effect of pH and temperature conditions on the proportion of basic isomeric antibodies are shown in FIG. 5C, and specifically, it was found that pH and temperature changes have a significant effect on the proportion of basic isomeric antibodies, and in particular, it was confirmed that pH conditions have a greater effect on the proportion of basic isomeric antibodies than temperature conditions. In addition, it was confirmed that within a certain range, the lower the pH and the higher the temperature, the lower the proportion of basic isomeric antibodies (FIG. 5C).

### Example 3-4: Analysis of changes in proportions of main active antibody and isomeric antibody according to lactic acid addition

In Example 1, changes in the proportions of main active antibodies and isomeric antibodies when 100 µl of 1 M L-lactic acid solution was injected every day from the 7th day to the 11th day of culture were analyzed in more detail through LapChip^{®}, and the results are shown in FIGS. 6A to 6C.

Specifically, in the case of the main active antibody, when lactic acid was added, it was observed that the antibody proportion was increased in both the CS1 group that was subjected to a culture temperature change and the CS2 group that was not subjected to a culture temperature change, and it was found that the antibody proportion increased about 1.5% to 6.6% depending on the pH conditions (FIG. 6A).

In the case of the acidic isomeric antibody, when lactic acid was added, an increase in the antibody proportion of about 0.6% to 3.1% was observed depending on pH conditions in the CS1 group that was subjected to a culture temperature change, and in the CS2 group that was not subjected to a culture temperature change, it was observed that the antibody proportion increased about 0.9% or decreased about 0.8% to 2.1% depending on the pH conditions (FIG. 6B).

In the case of the basic isomeric antibody, when lactic acid was added, it was observed that the antibody proportion decreased in both the CS1 group that was subjected to a culture temperature change and the CS2 group that was not subjected to a culture temperature change, and it was found that the antibody proportion decreased about 2.1% to 4.7% depending on the pH conditions (FIG. 6C).

### Example 3-5: Comprehensive analytical results of main active antibody and isomeric antibody proportions

Summarizing the results of the charge variant analysis using LapChip^{®} and CE-HPLC equipment according to Examples 3-1 to 3-4, it was confirmed that the advantageous conditions for obtaining an antibody population of quality(proportions of main active antibodies and isomeric antibodies) similar to that of a reference drug were conditions of a pH around 7.0, conditions of applying a temperature change from the initial temperature of 37 °C to 35 °C, and conditions of adding lactic acid.

### Example 4. N-glycan analysis

Among the antibody populations obtained in Example 1, N-glycan analysis was performed on the CS1 group that was subjected to a temperature change on the 5th day of culture, and the results are shown in FIG. 7. Specifically, FIG. 7A is a table showing the results of the N-glycan analysis and similarity analysis with the reference drug (Humira), and FIG. 7B schematically shows the glycan structures used in glycan analysis.

As a result of the N-glycan analysis, when focusing on the G0F, GOF-N, and GOF+GN forms among the glycan structures, it was found that under conditions of pH 6.9, when lactic acid was not added, 60.87% of the G0F form, 6.71% of the GOF-N form, and 9.13% of the GOF+GN form were included, and when lactic acid was added, 63.05% of the G0F form, 6.73% of the GOF-N form, and 8.38% of the GOF+GN form were included. The afucosylation results were analyzed to be 7.04% and 6.71%, respectively, and the galactosylation results were analyzed to be 80.70% and 82.31%, respectively.

It was found that under conditions of pH 7.0, when lactic acid was not added, 72.41% of the G0F form, 4.51% of the GOF-N form, and 4.37% of the GOF+GN form were included, and when lactic acid was added, 70.23% of the G0F form, 5.71% of the GOF-N form, and 4.39% of the GOF+GN form were included. The afucosylation results were analyzed to be 4.72% and 4.87%, respectively, and the galactosylation results were analyzed to be 84.52% and 85.47%, respectively.

It was found that under conditions of pH 7.1, when lactic acid was not added, 71.25% of the G0F form, 4.35% of the GOF-N form, and 4.39% of the GOF+GN form were included, and when lactic acid was added, 76.07% of the G0F form, 3.76% of the GOF-N form, and 3.31% of the GOF+GN form were included. The afucosylation results were analyzed to be 5.12% and 4.88%, respectively, and the galactosylation results were analyzed to be 83.39% and 86.60%, respectively.

As a result of comparing the above results with the glycan structure of the reference drug Humira ("HE-4" in FIG. 7A), it was found that the G0F, GOF-N, and GOF+GN forms were very similar to or slightly different from the reference drug under conditions of pH 7.0 and 7.1, and it was found that the results of afucosylation and galactosylation were also similar.

Comprehensively considering the results of Examples 1 to 4, summarizing the results of the IgG titer measurement, the charge variant analysis, and the N-glycan analysis of the obtained adalimumab antibody population, it was found that conditions of pH 7.0 to 7.1, especially pH 7.0, and conditions of applying a temperature change of lowering the culture temperature from the initial temperature of 37 °C to 35 °C, and conditions of adding lactic acid were advantageous for obtaining an antibody population of similar quality to that of the reference drug.

In the present specification, detailed description of contents that can be sufficiently recognized and inferred by those skilled in the technical field to which the present invention pertains is omitted, and in addition to the specific examples described in the present specification, various modifications are possible without changing the technical idea or essential features of the present invention. Therefore, the present invention may be implemented in ways other than those specifically described and exemplified in the present specification, which can be understood by those skilled in the technical field to which the present invention pertains

## Claims

1. A method for producing an antibody population, comprising:
(a) a first temperature culture step of culturing recombinant cells expressing an antibody in a medium under conditions of pH 7.0 to 7.1 at a first culture temperature of 36 °C to 38 °C for four to six days; and
(b) a second temperature culture step of culturing the cultured recombinant cells in a medium under conditions of pH 7.0 to 7.1 at a second culture temperature set to be 2 °C to 4 °C lower than the first culture temperature of the step (a), but higher than or equal to 34 °C for six to eight days.

2. The method according to claim 1, wherein the step (b) is performed by adding 80 to 120 µl of 0.1 to 2 M lactic acid per 10 ml of a medium every day from the first day, second day or third day of culture at the second culture temperature.

3. The method according to claim 1, wherein the antibody is a monoclonal antibody.

4. The method according to claim 1, wherein the antibody is adalimumab.

5. The method according to claim 1, wherein the antibody population includes 55% or more of the main active antibody and 45% or less of the isomeric antibody.

6. The method according to claim 1, wherein the antibody population includes 60% or more of the main active antibody and 40% or less of the isomeric antibody.

7. The method according to claim 1, wherein the antibodies of the antibody population have a glycan structure including 65% to 80% of a G0F form.

8. The method according to claim 1, wherein the antibodies of the antibody population have a glycan structure including 3% to 6% of a GOF+GN form.

9. A method for producing an antibody population, comprising:
(a) a first temperature culture step of culturing recombinant cells expressing an antibody in a culture medium under conditions of 36.5 °C to 37.5 °C and pH 7.0 to 7.1 for five days; and
(b) a second temperature culture step of culturing the cultured recombinant cells at a culture temperature of 34.5 °C to 35.5 °C under conditions of pH 7.0 to 7.1 for seven days while adding 95 to 105 µl of 0.5 to 2 M lactic acid per 10 ml of a medium every day from the third day of culture.

10. The method according to claim 9, wherein the antibody is adalimumab.
